# EUROPEAN PATENT APPLICATION

(11) **EP 4 582 123 A1**
(43) Date of publication of application: **09.07.2025**
(21) Application number: 24150607.0
(22) Date of filing: 08.01.2024
(51) Int. Cl.: A61M 16/00, A61M 11/00, A61M 16/06, A61M 16/16, A61B 5/08

(54) **CONTINUOUS POSITIVE AIRWAY PRESSURE CPAP DEVICE WITH NASAL CONGESTION DETECTION/DEVICE SETTINGS ADAPTATION AND METHOD THEREOF**

(71) Applicant: Koninklijke Philips N.V., 5656 AG Eindhoven (NL)
(72) Inventor: LOWET, Dietwig Jos Clement, Eindhoven (NL); KRANS, Jan Martijn, Eindhoven (NL)
(74) Representative: Philips Intellectual Property & Standards

(57) **Abstract**

A continuous positive airway pressure CPAP device (10) for providing a pressurized flow of breathable gas to a subject (20) during a sleep disordered breathing treatment comprises a blower (14), a nasal congestion detection module (42), and a device settings adaptation module (44). The blower (14) delivers the pressurized flow via a patient circuit (16) to a patient interface device (18) worn by the subject. The nasal congestion detection module (42) detects a presence of a nasal congestion condition of the subject based upon a combination of input parameters available to the CPAP device. The combination of input parameters is determined at least a predetermined time before delivery, or during delivery, of the pressurized flow of breathable gas. The device settings adaptation module (44) adapts at least one CPAP device setting based on the detection of the condition indicative of nasal congestion for reducing or alleviating nasal congestion during the sleep disordered breathing treatment.

## Description

### BACKGROUND

The present embodiments relate generally to positive airway pressure devices and more particularly, to a continuous positive airway pressure (CPAP) device featured with nasal congestion detection/device settings adaptation and a method thereof.

Obstructive sleep apnea (OSA) patients often use positive airway pressure (PAP) sleep therapy devices such as the Philips Dreamstation^{™} for example. Such PAP therapy devices make use of a patient interface device or facial mask connected via a patient interface circuit or tube to a base station which controls the flow of breathable gas and/or air pressure. The base station is typically located next to the patient's bed during use. Therapy settings of the positive airway pressure device can be read and tuned via a user interface on the base station. One form of a PAP device is a continuous positive airway pressure or CPAP device.

In general, CPAP devices utilize two different types of masks: full-face masks (FFM) and nasal masks. Nasal masks are considered more comfortable, but cannot be used when a person breathes through his or her mouth. Some patients are habitual breathing through their mouth and need to use a full-face mask. However, even people that habitually breathe through their nose will start breathing through their mouth when they have a cold or an allergy and their nose is congested. This leads to discomfort and non-adherence to CPAP therapy.

In particular, nasal congestion makes using CPAP uncomfortable or even impossible. In other words, nasal congestion leads to reduce adherence to therapy. Nasal congestion thus requires the patient to take active action, which is often not done at night, which is typically the time that a patient receives CPAP therapy. Accordingly, it would be desirable to provide a more automated way to reduce nasal congestion when using CPAP therapy.

To address issues of nasal congestion, some CPAP devices are provided with a humidifier. The humidifier introduces moisturize into the pressurized flow of breathable gas when the humidifier is powered during CPAP therapy. However, use of the humidifier can become uncomfortable for the CPAP user because the humidifier is either on or off during the CPAP therapy session, without any control of being turned on only when needed during a given therapy session. Furthermore, some CPAP device users prefer a facial mask, while others prefer a nasal mask. However, advice as to which mask type to use for a specific night can also depend on whether or not the user's nose is congested.

Accordingly, an improved method and apparatus for overcoming the problems in the art are desired. It would be desirable to provide a method and apparatus to detect nasal congestion for CPAP patients using a nasal mask and adapt CPAP device settings to alleviate the congestion.

### SUMMARY

One object of the present disclosure is to provide a method to detect nasal congestion for patients using a CPAP device with a nasal mask and, in response to the detected nasal congestion, adapt settings on the CPAP device to alleviate the congestion. Adapting CPAP device settings includes one or more of turning on a humidifier and adding decongestant agents to the pressurized flow of breathable gas.

According to one embodiment, a continuous positive airway pressure CPAP device for providing a pressurized flow of breathable gas to a subject during a sleep disordered breathing treatment comprises a blower, a nasal congestion detection module, and a device settings adaptation module. The blower is adapted to deliver the pressurized flow of breathable gas via a patient circuit to a patient interface device. The patient interface device is to be worn by the subject during the sleep disordered breathing treatment. The patient interface device can comprise, for example, a nasal mask. The nasal congestion detection module is adapted to detect a presence of a nasal congestion condition of the subject based upon a combination of input parameters available to the CPAP device. The combination of input parameters is determined at least a predetermined time before delivery, or during delivery, of the pressurized flow of breathable gas. The device settings adaptation module is configured to adapt at least one CPAP device setting based on the detection of the condition indicative of nasal congestion. The adaptation of the at least one CPAP device setting contributes to reducing or alleviating (or is operative to reduce or alleviate) nasal congestion during the sleep disordered breathing treatment.

According to another embodiment, the CPAP device includes wherein the nasal congestion detection module comprises at least one (or at least two) selected from the group consisting of: (i) a voice analysis module, (ii) a mouth breathing detection module, and (iii) a CPAP usage tracking module. The voice analysis module is adapted to detect the nasal congestion condition from a first input parameter based on an analysis of a voice signal of the subject. The voice signal is captured via a microphone coupled to the CPAP device. The microphone may comprise a microphone integral with the CPAP device, a microphone remote from the CPAP device, or a combination of both a microphone integral with and a microphone remote from the CPAP device. The mouth breathing detection module is adapted to detect the nasal congestion condition from a second input parameter based on an analysis of a measured air flow signal (e.g., indicative of either one of a nose breathing condition or a mouth breathing condition or a combination thereof). An unexpected mouth breathing condition determined via the analysis of the measured air flow signal is indicative of the nasal congestion. The CPAP usage tracking module is adapted to detect the nasal congestion condition from a third input parameter based on a reduced CPAP usage (i.e., by the subject) greater than a predetermined CPAP usage over a threshold period of time. The reduced CPAP usage is based on at least one of (a) a measurement of patient interface device wear time ON and wear time OFF (e.g., during an episode of sleep disordered breathing treatment) and (b) a measurement of sleep disordered breathing treatment continuous use time and discontinuous use time.

In accordance with another embodiment, the CPAP device further comprises a decongestant agent dispenser configured to dispense a predetermined quantity of at least one nose decongestant agent selected from a predetermined group of nose decongestant agents. In addition, the at least one CPAP device setting may comprise at least a decongestant agent dispenser setting. The decongestant agent dispenser setting is normally set to OFF in an absence of the detection of the condition indicative of nasal congestion. In the OFF setting, the decongestant agent dispenser does not dispense the predetermined quantity of the at least one nose decongestant agent into the pressurized flow of breathable gas. The decongestant agent dispenser setting is set to ON only in response to the detection of the condition indicative of nasal congestion. In the ON setting, the decongestant agent dispenser dispenses the predetermined quantity of the at least one nose decongestant agent into the pressurized flow of breathable gas. The predetermined group of nose decongestant agents may comprise non-medical agents selected from the group consisting of menthol, eucalyptus, and saline spray.

In yet another embodiment, the CPAP device further comprises a humidifier configured to humidify the pressurized flow of breathable gas according to a predetermined humidification level, wherein the at least one CPAP device setting may comprise a humidifier setting. The humidifier setting is normally set to OFF in an absence of the detection of the condition indicative of nasal congestion. In the OFF setting, the humidifier does not moisten the pressurized flow of breathable gas. The humidifier setting is set to ON only in response to the detection of the condition indicative of nasal congestion. In the ON setting, the humidifier moistens the pressurized flow of breathable gas according to the predetermined humidification level.

In another embodiment, the CPAP device further comprises a head position adapter configured to tilt a subject's head towards a more vertical position from a more horizontal position, wherein the at least one CPAP device setting may comprise a head position adapter setting. The head position adapter comprises one or more of (i) an inflatable head pillow adapted to inflate/deflate between the more horizontal position and the more vertical position and (ii) a tiltable mattress adapted to tilt between the more horizontal position and the more vertical position. The head position adapter setting is normally set to the horizontal position corresponding to an absence of the detection of the condition indicative of nasal congestion. The head position adapter setting is set to the vertical position only in response to the detection of the condition indicative of nasal congestion. In the vertical position setting, the head position adapter inflates the inflatable head pillow to a corresponding predetermined vertical pillow position or tilts the tiltable mattress to a corresponding predetermined vertical mattress position.

According to another embodiment, the CPAP device further comprises a patient interface type recommendation module. The patient interface type recommendation module is configured to provide a mask type recommendation. The patient interface type recommendation module provides the recommendation via an input/output device of the CPAP device in response to a continued detection of the condition indicative of nasal congestion for a period of time greater than a predetermined threshold period of time for at least a single episode of the sleep disordered breathing treatment. The mask type recommendation communicated to the subject is configured to advise the subject to wear a mask type other than an only nasal mask type.

In accordance with one embodiment, a method for providing a pressurized flow of breathable gas to a subject during a sleep disordered breathing treatment via a continuous positive airway pressure CPAP device comprises: delivering the pressurized flow of breathable gas, detecting a nasal congestion condition, and adapting at least one CPAP device setting based on the detection of the condition indicative of nasal congestion. Delivering the pressurized flow of breathable gas includes delivering, via a blower and a patient circuit, to a patient interface device to be worn by the subject during the sleep disordered breathing treatment. The patient interface device can comprise, for example, a nasal mask. Detecting the nasal congestion condition includes detecting, via a nasal congestion detection module, a presence of a nasal congestion condition of the subject based upon a combination of input parameters available to the CPAP device. The combination of input parameters is determined at least a predetermined time before delivery, or during delivery, of the pressurized flow of breathable gas. Adapting the at least one CPAP device setting includes adapting, via a device settings adaptation module, at least one CPAP device setting based on the detection of the condition indicative of nasal congestion. The adaptation of the at least one CPAP device setting contributes to reducing or alleviating (or is operative to reduce or alleviate) nasal congestion during the sleep disordered breathing treatment.

According to another embodiment, the method includes wherein detecting the nasal congestion condition, via the nasal congestion detection module, comprises at least one (or at least two) selected from the group consisting of: (i) detecting via a voice analysis module, (ii) detecting via a mouth breathing detection module, and (iii) detecting via a CPAP usage tracking module. The voice analysis module is adapted to detect the nasal congestion condition from a first input parameter based on an analysis of a voice signal of the subject. The voice signal is captured via a microphone coupled to the CPAP device. The microphone may comprise a microphone integral with the CPAP device, a microphone remote from the CPAP device, or a combination of both a microphone integral with and a microphone remote from the CPAP device. The mouth breathing detection module is adapted to detect the nasal congestion condition from a second input parameter based on an analysis of a measured air flow signal (e.g., indicative of either one of a nose breathing condition or a mouth breathing condition or a combination thereof). An unexpected mouth breathing condition determined via the analysis of the measured air flow signal is indicative of the nasal congestion. The CPAP usage tracking module is adapted to detect the nasal congestion condition from a third input parameter based on a reduced CPAP usage (i.e., by the subject) greater than a predetermined CPAP usage over a threshold period of time. The reduced CPAP usage is based on at least one of (a) a measurement of patient interface device wear time ON and wear time OFF (e.g., during an episode of sleep disordered breathing treatment) and (b) a measurement of sleep disordered breathing treatment continuous use time and discontinuous use time.

In accordance with another embodiment, the method further comprises dispensing, via a decongestant agent dispenser, a predetermined quantity of at least one nose decongestant agent selected from a predetermined group of nose decongestant agents. The predetermined group of nose decongestant agents may comprise non-medical agents selected from the group consisting of menthol, eucalyptus, and saline spray. In addition, the at least one CPAP device setting may comprise at least a decongestant agent dispenser setting. The decongestant agent dispenser setting is normally set to OFF in an absence of the detection of the condition indicative of nasal congestion. In the OFF setting, the decongestant agent dispenser does not dispense the predetermined quantity of the at least one nose decongestant agent into the pressurized flow of breathable gas. The decongestant agent dispenser setting is set to ON only in response to the detection of the condition indicative of nasal congestion. In the ON setting, the decongestant agent dispenser dispenses the predetermined quantity of the at least one nose decongestant agent into the pressurized flow of breathable gas.

In yet another embodiment, the method further comprises humidifying, via a humidifier, the pressurized flow of breathable gas, wherein the at least one CPAP device setting comprises at least a humidifier setting. The humidifier setting is normally set to OFF in an absence of the detection of the condition indicative of nasal congestion. In the OFF setting, the humidifier does not moisten the pressurized flow of breathable gas. The humidifier setting is set to ON only in response to the detection of the condition indicative of nasal congestion. In the ON setting, the humidifier moistens the pressurized flow of breathable gas according to the predetermined humidification level.

In another embodiment, the method further comprises tilting, via a head position adapter, a subject's head towards a more vertical position from a more horizontal position, wherein the at least one CPAP device setting may comprise a head position adapter setting. The head position adapter comprises one or more of (i) an inflatable head pillow adapted to inflate/deflate between the more horizontal position and the more vertical position and (ii) a tiltable mattress adapted to tilt between the more horizontal position and the more vertical position. The head position adapter setting is normally set to the horizontal position corresponding to an absence of the detection of the condition indicative of nasal congestion. The head position adapter setting is set to the vertical position only in response to the detection of the condition indicative of nasal congestion. In the vertical position setting, the head position adapter inflates the inflatable head pillow to a corresponding predetermined vertical pillow position or tilts the tiltable mattress to a corresponding predetermined vertical mattress position.

According to another embodiment, the method further comprises providing, via a patient interface type recommendation module and an input/output device of the CPAP device, a mask type recommendation. The patient interface type recommendation module provides the recommendation via an input/output device of the CPAP device in response to a continued detection of the condition indicative of nasal congestion for a period of time greater than a predetermined threshold period of time for at least a single episode of the sleep disordered breathing treatment. The mask type recommendation to be communicated to the subject is configured to advise the subject to wear a mask type other than an only nasal mask type.

The embodiments of the present disclosure advantageously solve or overcome the problems in the art by providing a method and apparatus to detect nasal congestion for CPAP patients using a nasal mask and adapting, in response to detecting nasal congestion, CPAP device settings to alleviate the congestion. Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing Figures, like reference numerals refer to like elements. In addition, it is to be noted that the Figures may not be drawn to scale.
Fig. 1 is a block diagram view of a continuous positive airway pressure (CPAP) device with nasal congestion detection and device settings adaptation according to an embodiment of the present disclosure;
Fig. 2 is a block diagram view of the controller of the CPAP device with nasal congestion detection and device settings adaptation of Fig. 1 in further detail according to an embodiment of the present disclosure;
Fig. 3 is a flow diagram view of nasal congestion detection and device settings adaptation in the CPAP device according to an embodiment of the present disclosure;
Fig. 4 is a graphical representation view of normal nose breathing and mouth breathing with respect to time in connection with a nasal congestion detection module of the CPAP device with nasal congestion detection and device settings adaptation according to an embodiment of the present disclosure;
Fig. 5 is a perspective view of a patient interface device of the CPAP device with nasal congestion detection and device settings adaptation, according to an embodiment of the present disclosure;
Fig. 6 is a perspective view of a patient interface device of the CPAP device with nasal congestion detection and device settings adaptation, in the form of a nasal mask with an attached patient circuit, according to another embodiment of the present disclosure; and
Fig. 7 is a flow diagram view of a method for providing a pressurized flow of breathable gas using a continuous positive airway pressure CPAP device with nasal congestion detection and device settings adaptation according to an embodiment of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

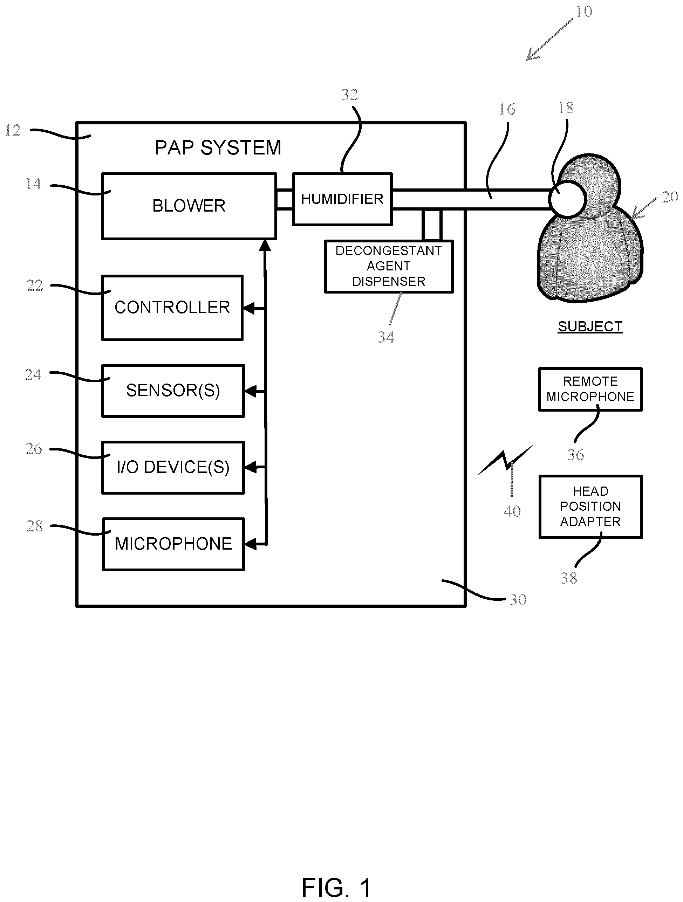

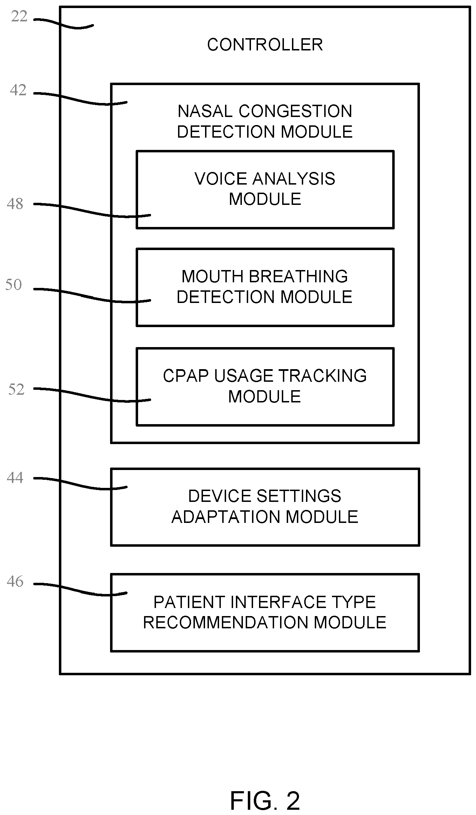

With reference to Fig. 1, there is shown a block diagram view of a continuous positive airway pressure (CPAP) system or device 10 with nasal congestion detection and device settings adaptation according to an embodiment of the present disclosure. The CPAP system 10 comprises a positive airway pressure device 12 having a gas flow generator or blower 14 configured to generate a pressurized flow of breathable gas. The gas flow generator 14 is configured to communicate the pressurized flow of breathable gas to a patient circuit 16 that includes a patient interface device or mask 18 to a patient or subject 20. The system further includes a controller 22 configured to implement one or more modules, as will be explained with reference to Fig. 2. In one embodiment, controller 22 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given PAP system implementation and/or application.

Controller 22 can further comprise one or more various modules configured to implement a given functionality as discussed herein. For example, the modules may comprise one or more of an integrated circuit, discrete analog or digital circuit components, hardware, software, firmware, or any combination thereof, for performing various functions as discussed herein, further according to the requirements of a given nasal congestion detection and CPAP device settings adaptation implementation and/or application according to an embodiment of the present disclosure. In addition, one or more of the modules may further comprise various combinations of one or more of the various modules as discussed herein. Furthermore, it is understood that the described modules may be computer program modules which are rendered in a non-transitory computer-readable medium.

One or more sensor 24 (or sensors) are provided and configured to generate output signals related to at least one characteristic associated with at least one of respiratory, actigraphy, airflow, humidity, pressure, temperature, in-mask sensor signals, or any combination thereof. First sensor signals may comprise respiratory signals, actigraphy signals, or a combination of both. Second sensor signals may comprise airflow sensor signals, pressure sensor signals, in-mask sensor signals, IR sensor signals (e.g., to detect a distance between a PAP mask and a user's forehead), or a combination thereof.

One or more input/output (I/O) device(s), collectively indicated by reference numeral 26, is representative of any number of suitable I/O devices for inputting or outputting one or more of an audible, visual, and/or tactile input or output, according to the requirements of a given PAP system implementation with nasal congestion detection and device settings adaptation, as discussed herein. For example, input/output device(s) 26 can comprise one or more of an input/output device, a user interface, tactile output device, touch screen, optical display, microphone (e.g., for receiving user voice commands/responses), keypad, keyboard, pointing device, image capture device, video camera, audio output device, and any combination thereof, according to the requirements of the given PAP system implementation with nasal congestion detection and device settings adaptation. In another embodiment, input/output device 26 may include a mobile phone app configured for inputting or outputting one or more of an audible, visual, and/or tactile input or output, according to the requirements of a given PAP system implementation with nasal congestion detection and device settings adaptation.

With reference still to Fig. 1, the continuous positive airway pressure (CPAP) device 10 with nasal congestion detection and device settings adaptation further comprises a dedicated nasal congestion detection microphone 28 located within base station housing 30, a humidifier 32, a decongestant agent dispenser 34, a remote microphone 36, and a head position adapter 38, as will be discussed further herein. In Fig. 1, the lightning bolt identified via reference numeral 40 is indicative of a wired or wireless link (e.g., near field communications, Bluetooth^{™} or other wireless communications link) between a given component (e.g., controller 22) of the PAP system 12 and the remote microphone 36 and/or head position adapter 38, as will be discussed further herein.

Referring now to Fig. 2, there is shown a block diagram view of the controller 22 of the CPAP device with nasal congestion detection and device settings adaptation of Fig. 1 in further detail. In one embodiment, controller 22 comprises a nasal congestion detection module 42 and a device settings adaptation module 44. In one embodiment, controller 22 may further comprise a patient interface type recommendation module 46, as will be discussed further herein. The nasal congestion detection module 42 is adapted to detect a presence of a nasal congestion condition of the subject based upon a combination of input parameters available to the CPAP device. The combination of input parameters is determined at least a predetermined time before delivery, or during delivery, of the pressurized flow of breathable gas. The device settings adaptation module 44 is configured to adapt at least one CPAP device setting based on the detection of the condition indicative of nasal congestion. The adaptation of the at least one CPAP device setting contributes to reducing or alleviating (or is operative to reduce or alleviate) nasal congestion during the sleep disordered breathing treatment or therapy.

In one embodiment, the nasal congestion detection module 42 comprises at least one (or at least two) selected from the group consisting of: (i) a voice analysis module 48, (ii) a mouth breathing detection module 50, and (iii) a CPAP usage tracking module 52. The voice analysis module 48 is adapted to detect the nasal congestion condition from a first input parameter based on an analysis of a voice signal of the subject. The voice signal is captured via a microphone coupled to the CPAP device 10. The microphone may comprise a microphone 28 integral with the CPAP device, a microphone 36 remote from the CPAP device, or a combination of both a microphone 28 integral with and a microphone 36 remote from the CPAP device.

The mouth breathing detection module 50 is adapted to detect the nasal congestion condition from a second input parameter based on an analysis of a measured air flow signal (e.g., indicative of either one of a nose breathing condition or a mouth breathing condition or a combination thereof). An unexpected mouth breathing condition determined via the analysis of the measured air flow signal can be indicative of the nasal congestion. The CPAP usage tracking module 52 is adapted to detect the nasal congestion condition from a third input parameter based on a reduced CPAP usage (i.e., by the subject) greater than a predetermined CPAP usage over a threshold period of time. The reduced CPAP usage is based on at least one of (a) a measurement of patient interface device 18 wear time ON and wear time OFF (e.g., during an episode of sleep disordered breathing treatment or therapy) and (b) a measurement of sleep disordered breathing treatment continuous use time and discontinuous use time (e.g., per treatment or therapy episode or for a given plurality of treatments or episodes).

With reference now to Figs 1 and 2, the device settings adaptation module 44 is configured to adapt at least one CPAP device setting based on the detection of the condition indicative of nasal congestion. The adaptation of the at least one CPAP device setting contributes to reducing or alleviating (or is operative to reduce or alleviate) nasal congestion during the sleep disordered breathing treatment or therapy. In one embodiment, the CPAP device 10 comprises the humidifier 32. Humidifier 32 is configured to humidify the pressurized flow of breathable gas according to a predetermined humidification level, wherein the at least one CPAP device setting may comprise a humidifier setting. The humidifier setting is normally set to OFF in an absence of the detection of the condition indicative of nasal congestion. In the OFF setting, the humidifier does not moisten the pressurized flow of breathable gas. The humidifier setting is set to ON only in response to the detection of the condition indicative of nasal congestion. In the ON setting, the humidifier moistens the pressurized flow of breathable gas according to the predetermined humidification level.

In another embodiment, the CPAP device 10 comprises the decongestant agent dispenser 34. Decongestant agent dispenser 34 is configured to dispense a predetermined quantity of at least one nose decongestant agent selected from a predetermined group of nose decongestant agents. In addition, the at least one CPAP device setting may comprise at least a decongestant agent dispenser setting. The decongestant agent dispenser setting is normally set to OFF in an absence of the detection of the condition indicative of nasal congestion. In the OFF setting, the decongestant agent dispenser does not dispense the predetermined quantity of the at least one nose decongestant agent into the pressurized flow of breathable gas. The decongestant agent dispenser setting is set to ON only in response to the detection of the condition indicative of nasal congestion. In the ON setting, the decongestant agent dispenser dispenses the predetermined quantity of the at least one nose decongestant agent into the pressurized flow of breathable gas. The predetermined group of nose decongestant agents may comprise non-medical agents selected from the group consisting of menthol, eucalyptus, and saline spray.

In another embodiment, the CPAP device 12 further comprises the head position adapter 38. The head position adapter 38 is configured to tilt a subject's head towards a more vertical position from a more horizontal position, wherein the at least one CPAP device setting may comprise a head position adapter setting. The head position adapter 38 may comprises one or more of (i) an inflatable head pillow adapted to inflate/deflate between the more horizontal position and the more vertical position and (ii) a tiltable mattress adapted to tilt between the more horizontal position and the more vertical position. The head position adapter setting is normally set to the horizontal position corresponding to an absence of the detection of the condition indicative of nasal congestion. The head position adapter setting is set to the vertical position only in response to the detection of the condition indicative of nasal congestion. In the vertical position setting, the head position adapter 38 inflates the inflatable head pillow to a corresponding predetermined vertical pillow position or tilts the tiltable mattress to a corresponding predetermined vertical mattress position.

With reference to Fig. 2, the CPAP device 10 further comprises the patient interface type recommendation module 46. The patient interface type recommendation module 46 is configured to provide a mask type recommendation. The patient interface type recommendation module 46 provides the recommendation via an input/output device 26 of the CPAP device 10 in response to a continued detection of the condition indicative of nasal congestion for a period of time greater than a predetermined threshold period of time for at least a single episode of the sleep disordered breathing treatment. The mask type recommendation communicated to the subject 20 is configured to advise the subject to wear a mask type other than an only nasal mask type.

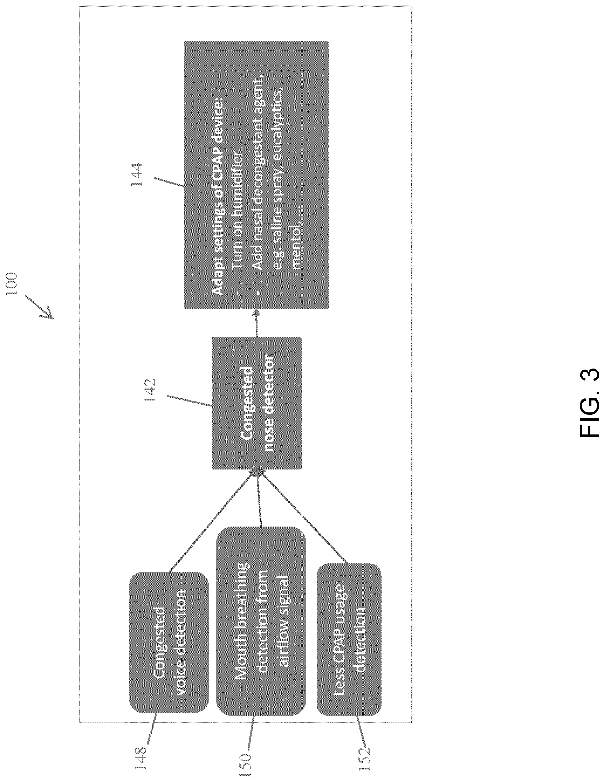

With reference now to Fig. 3, there is shown a flow diagram 100 of nasal congestion detection and device settings adaptation in the CPAP device 10 according to an embodiment of the present disclosure. The flow diagram 100 illustrates aspects of an automated measurement and/or detection of congestive symptoms by the CPAP device 10. Nasal congestion can be detected by the CPAP device 10 via the following elements as inputs to a congested nose detector 142: (i) congested voice detection 148, (ii) mouth breathing detection 150, and (iii) CPAP usage detection 152. With respect to congested voice detection 148, a suitable voice analysis (i.e., comparing a normal voice with a congested voice) is performed, via controller 22, based upon voice recordings captured via a voice interface (e.g., microphone 28, remote microphone 36, or both) for ease of interaction with the CPAP device 10. With respect to mouth breathing detection 150, CPAP mouth breathing detection is determined, via controller 22, based on a measured air flow signal, via sensor(s) 24. For example, an unexpected mouth breathing can be a strong indicator of nasal congestion. With respect to CPAP usage detection 152, nasal congestion typically leads to a patient taking his or her mask off and not continuing the CPAP treatment for the duration of a given episode or for one or more therapy session(s). A detection of less CPAP usage based upon a given history of greater CPAP usage may be indicative of nasal congestion. In other words, a determination of less CPAP usage over a given period of time may be used as an indicator of nasal congestion. Nasal congestion is typically something that lasts multiple days. Therefore, less CPAP usage from previous days can be used as a potential indicator of nasal congestion.

Accordingly, congested nose detector 142 receives input from (i) the congested voice detection 148, (ii) the mouth breathing detection 150, and (iii) the CPAP usage detection 152. The output of the congested nose detector 142 provides input to an adapter 142 configured to adapt settings of the CPAP device. With reference still to Fig. 3, in to detection of a congested nose condition or nose congestion via the congested nose detector 142, the CPAP device settings are adapted to (i) automatically turn on the humidifier 32 to humidify the pressurized flow of breathable gas only when needed (i.e., in response to detecting nose congestion), and (ii) automatically adding or administering nose decongestive agents to the pressurized flow of breathable gas (e.g., menthol, eucalyptus, etc.).

As illustrated in the flow diagram 100 of Fig. 3, detection of nasal congestion can be accomplished by combining a number of input parameters available to the CPAP device 10. With respect to voice detection 148, voice analysis is carried out via a voice interface implemented, e.g., via controller 22, I/O device(s) 26, microphone 28 and/or remote microphone 36 at the CPAP device 12. The voice interface can be configured to receive user prompts and/or instructions for initiating operation of and/or other aspects of operating the CPAP device 12, and/or to get motivational therapy advice, therapy statistics, etc. For example, if the subject or patient uses the CPAP device regularly, voice characteristics of the subject can be compared over time. A profile of what the subject's normal voice sounds like can be made, recorded, stored, and/or analyzed. When the voice of the user changes and starts having the typical sound characteristics of a congested person, the corresponding congested nasal condition can be detected. In other words, the CPAP device 12 can be configured for building a normal voice profile over time for the subject. The normal voice profile could then be used as a reference for use in detecting the congested nasal condition. For example, every evening, the CPAP device 10 may be configured to check for deviations in voice characteristics and determine whether those deviations are characteristic of nasal congestion.

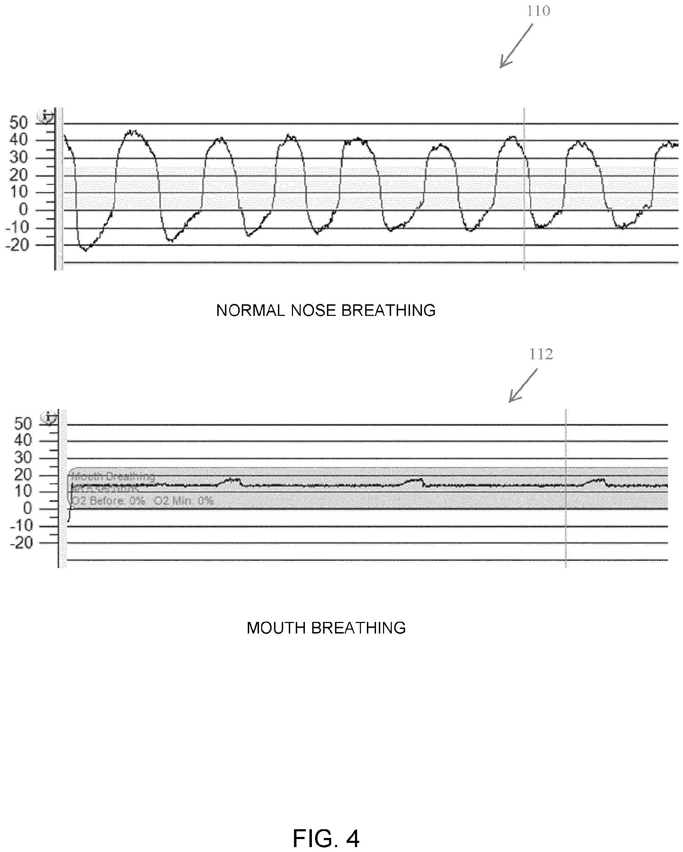

As indicated herein above, detection of nasal congestion can be accomplished by detecting mouth breathing from an airflow signal. Mouth breathing can be relatively easily detected in nasal CPAP masks since less breathing is detected. With reference now to Fig. 4, there is illustrated a graphical representation view of normal nose or nasal breathing 110 and mouth breathing 112 for a subject receiving CPAP treatment using a nasal mask (i.e., patient interface device 18) with respect to time. Mouth breathing detection is accomplished in connection with the nasal congestion detection module 42, mouth breathing detection module 50, and mouth breathing detection 150 of the CPAP device 10. As shown in Fig. 4, there exists measurable characteristics based on the typical difference in measured airflow signal between a person breathing through his/her nose (i.e., nasal breathing 110) and a person breathing through his/her mouth (i.e., mouth breathing 112).

In one embodiment, the microphone 28 integrated in the base station 30 of the CPAP therapy device 10 could be used to detect the user sounds during talking and breathing. By analysis of this detected audio signal, CPAP therapy device 10 can be configured to detect whether or not the user has a congested nasal sound when interacting with the CPAP therapy device 10, and/or the CPAP therapy device 10 can be configured to detect whether the user produces a breathing sound during sleep that indicates either mouth or nasal breathing. According to another embodiment, the remote microphone 36 may comprise a microphone of a mobile device or other bedside unit which could be used for sound detection purposes during interactions with the CPAP device 10, or during sleep, similarly as discussed with respect to the integrated microphone 28. In a further embodiment, the breathing sounds could also be detected by a remote microphone 36 integrated in or clipped on the patient interface device 18, i.e., nasal mask.

Adapting settings of the CPAP device 10 to alleviate nasal congestion may include one or more of turning on the humidifier 32, adding nose decongestant agents via decongestant agent dispenser 34, and adapting the subjects head position via head position adapter 38. In one embodiment, CPAP device 10 includes humidifier 32 to moisten the pressurized flow of breathable gas. This moistening of the breathable gas helps to prevent drying out of the nose, mouth and throat of the subject 20, and it also helps to prevent nasal congestion. However, the humidifier being turned ON for the duration of a therapy session is not always comfortable since it can lead to "rain-out" (which happens when the water in the air condenses, and the patient or subject ends up with water in the tube or patient interface circuit). In view of "rain-out", many patients do not use the humidifier feature of a CPAP device. However, according to the embodiments of the present disclosure, automated nose congestion detection may be used to turn on the humidifier only when needed, thereby making the humidifier much more effective.

With respect to nose decongestant agents, the embodiments of the present disclosure may include adding (non-medical) nose decongestant agents, via the decongestant agent dispenser 34, to the pressurized flow of breathable gas by the CPAP device 10. Examples of nose decongestant agents may include: Saline spray, Menthol, Eucalyptus, etc. In one embodiment, the added nose decongestant agents comprise a metered predetermined amount of at least one nose decongestant agent.

With respect to adapting a subject's head position, the embodiments of the present disclosure may be adapted to stimulate mucus to flow out of the nasal passages by tilting the head towards a more vertical position, from a more horizontal position. In one embodiment, this could also be realized by congested nose sound induced inflation of the head pillow, or tilting a mattress upon which the subject is disposed during the CPAP treatment or therapy. Adapting the subject's head position can be achieved via the head position adapter 38, as previously discussed.

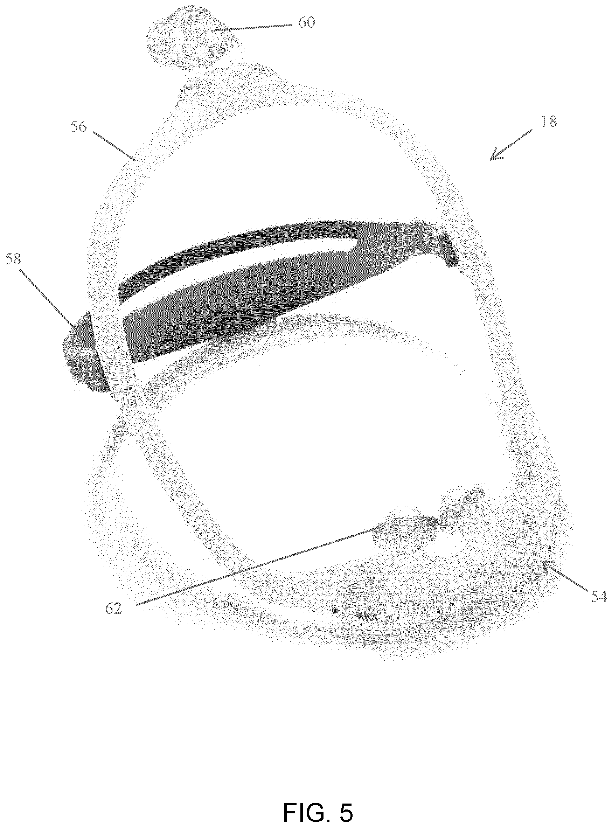

Turning now to Fig. 5, a perspective view is shown of a patient interface device 18 of the CPAP device 10 with nasal congestion detection and device settings adaptation, in the form of a nasal mask 54, according to an embodiment of the present disclosure. The nasal mask 54 is configured to direct airflow through the frame 56 (e.g., a soft silicone frame) so patients can wear glasses, watch TV and sleep comfortably. Mask 54 includes a headgear component 58 (e.g., one or more low-stretch headgear straps and/or arms of a soft, slip-resistant design that provide stability and a consistent fit) that physically couples to the nasal mask frame 56 via headgear adjustment tabs located at left and right sides of the frame. A patient circuit elbow connector 60 (i.e., tubing connection) is located at the top of the frame 56 (and thus on top of a user's head) which allows patients when wearing the mask to sleep in any position - stomach, side or back. Still further, the nasal mask 54 comprises a silicone pillows cushion mask that conforms to various nose shapes and sizes. The nasal mask 54 is an under-the-nose design. It features a silicone pillows cushion 62 which is soft to the touch, softer than gel pillows, which deliver an exceptional nostril seal.

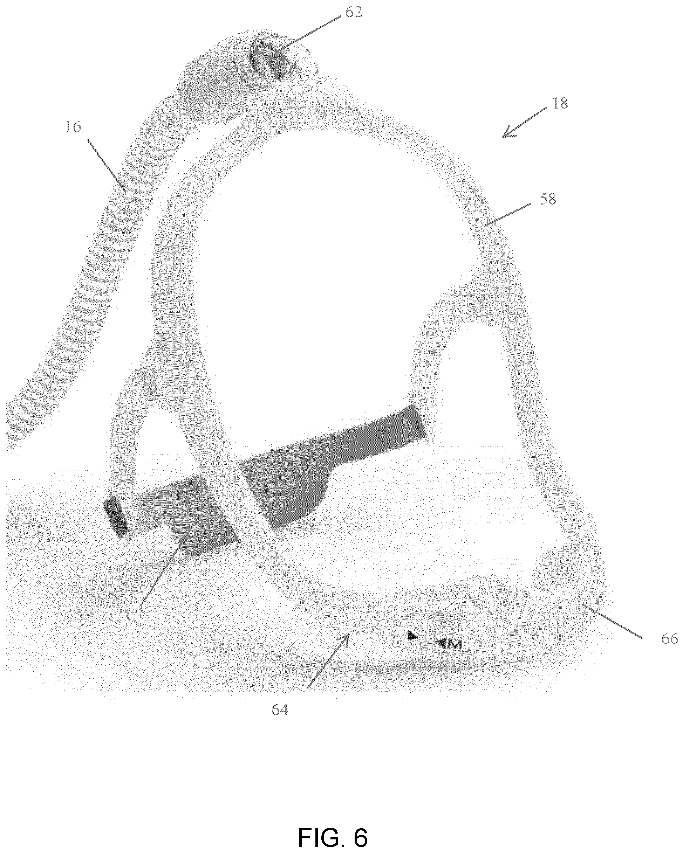

With reference now to Fig. 6, a perspective view is shown of a patient interface device 18 of the CPAP device 10 with nasal congestion detection and device settings adaptation, in the form of a nasal mask 64 with an attached patient circuit 16, according to another embodiment of the present disclosure. The embodiment of Fig. 6 is similar to that of Fig. 5, with the exception of an under-the-nose nasal cushion 66 in place of the nasal gel pillow cushion 62, according to an embodiment of the present disclosure. In this embodiment, the cushion opening is placed directly under the user's nostrils, whereby the cushion 66 hugs the user's nose and leaks will be minimal.

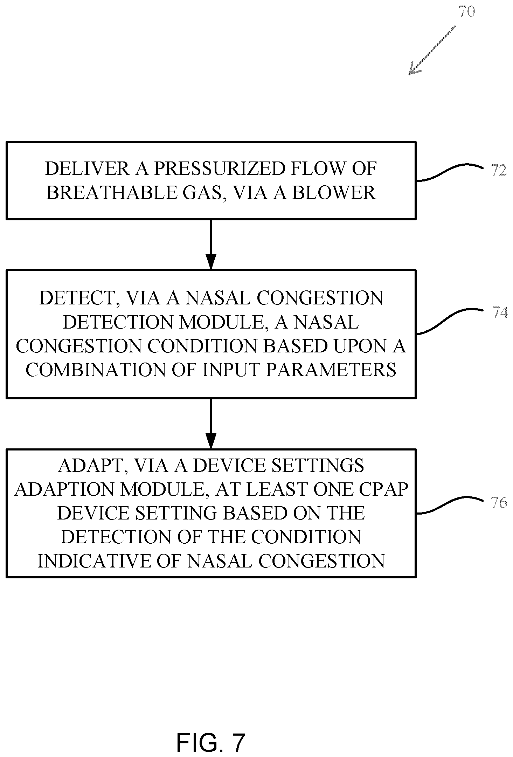

Turning now to Fig. 7, there is shown a flow diagram view of a method 70 for providing a pressurized flow of breathable gas to a subject during a sleep disordered breathing treatment via a continuous positive airway pressure CPAP device. A first step 72 includes delivering a pressurized flow of breathable gas to a subject. Delivering the pressurized flow of breathable gas includes delivering, via a blower and a patient circuit, to a patient interface device to be worn by the subject during the sleep disordered breathing treatment. The patient interface device can comprise, for example, a nasal mask.

A second step 74 includes detecting, via a nasal congestion detection module, a nasal congestion condition based upon a combination of input parameters. In particular, detecting the nasal congestion condition includes detecting, via the nasal congestion detection module, a presence of a nasal congestion condition of the subject based upon a combination of input parameters available to the CPAP device. The combination of input parameters is determined at least a predetermined time before delivery, or during delivery, of the pressurized flow of breathable gas.

According to one embodiment, detecting the nasal congestion condition, via the nasal congestion detection module, comprises at least one (or at least two) selected from the group consisting of: (i) detecting via a voice analysis module, (ii) detecting via a mouth breathing detection module, and (iii) detecting via a CPAP usage tracking module. The voice analysis module is adapted to detect the nasal congestion condition from a first input parameter based on an analysis of a voice signal of the subject. The voice signal is captured via a microphone coupled to the CPAP device. The microphone may comprise a microphone integral with the CPAP device, a microphone remote from the CPAP device, or a combination of both a microphone integral with and a microphone remote from the CPAP device. The mouth breathing detection module is adapted to detect the nasal congestion condition from a second input parameter based on an analysis of a measured air flow signal (e.g., indicative of either one of a nose breathing condition or a mouth breathing condition or a combination thereof). An unexpected mouth breathing condition determined via the analysis of the measured air flow signal is indicative of the nasal congestion. The CPAP usage tracking module is adapted to detect the nasal congestion condition from a third input parameter based on a reduced CPAP usage (i.e., by the subject) greater than a predetermined CPAP usage over a threshold period of time. The reduced CPAP usage is based on at least one of (a) a measurement of patient interface device wear time ON and wear time OFF (e.g., during an episode of sleep disordered breathing treatment) and (b) a measurement of sleep disordered breathing treatment continuous use time and discontinuous use time.

A third step 76 includes adapting at least one CPAP device setting based on the detection of the condition indicative of nasal congestion. More particularly, adapting the at least one CPAP device setting includes adapting, via a device settings adaptation module, at least one CPAP device setting based on the detection of the condition indicative of nasal congestion. The adaptation of the at least one CPAP device setting contributes to reducing or alleviating (or is operative to reduce or alleviate) nasal congestion during the sleep disordered breathing treatment, as previously discussed herein.

One example for adapting device settings in accordance with another embodiment includes wherein the method further comprises dispensing, via a decongestant agent dispenser, a predetermined quantity of at least one nose decongestant agent selected from a predetermined group of nose decongestant agents. The predetermined group of nose decongestant agents may comprise non-medical agents selected from the group consisting of menthol, eucalyptus, and saline spray. In addition, the at least one CPAP device setting may comprise at least a decongestant agent dispenser setting. The decongestant agent dispenser setting is normally set to OFF in an absence of the detection of the condition indicative of nasal congestion. In the OFF setting, the decongestant agent dispenser does not dispense the predetermined quantity of the at least one nose decongestant agent into the pressurized flow of breathable gas. The decongestant agent dispenser setting is set to ON only in response to the detection of the condition indicative of nasal congestion. In the ON setting, the decongestant agent dispenser dispenses the predetermined quantity of the at least one nose decongestant agent into the pressurized flow of breathable gas.

Another example for adapting device settings in yet another embodiment includes wherein the method further comprises humidifying, via a humidifier, the pressurized flow of breathable gas, wherein the at least one CPAP device setting comprises at least a humidifier setting. The humidifier setting is normally set to OFF in an absence of the detection of the condition indicative of nasal congestion. In the OFF setting, the humidifier does not moisten the pressurized flow of breathable gas. The humidifier setting is set to ON only in response to the detection of the condition indicative of nasal congestion. In the ON setting, the humidifier moistens the pressurized flow of breathable gas according to the predetermined humidification level.

Still another example for adapting device settings in another embodiment includes wherein the method further comprises tilting, via a head position adapter, a subject's head towards a more vertical position from a more horizontal position, wherein the at least one CPAP device setting may comprise a head position adapter setting. The head position adapter comprises one or more of (i) an inflatable head pillow adapted to inflate/deflate between the more horizontal position and the more vertical position and (ii) a tiltable mattress adapted to tilt between the more horizontal position and the more vertical position. The head position adapter setting is normally set to the horizontal position corresponding to an absence of the detection of the condition indicative of nasal congestion. The head position adapter setting is set to the vertical position only in response to the detection of the condition indicative of nasal congestion. In the vertical position setting, the head position adapter inflates the inflatable head pillow to a corresponding predetermined vertical pillow position or tilts the tiltable mattress to a corresponding predetermined vertical mattress position.

According to another embodiment, the method further comprises providing, via a patient interface type recommendation module and an input/output device of the CPAP device, a mask type recommendation. The patient interface type recommendation module provides the recommendation via an input/output device of the CPAP device in response to a continued detection of the condition indicative of nasal congestion for a period of time greater than a predetermined threshold period of time for at least a single episode of the sleep disordered breathing treatment. The mask type recommendation to be communicated to the subject is configured to advise the subject to wear a mask type other than an only nasal mask type.

Although only a few exemplary embodiments have been described in detail above, those skilled in the art will readily appreciate that many modifications are possible in the exemplary embodiments without materially departing from the novel teachings and advantages of the embodiments of the present disclosure. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A continuous positive airway pressure CPAP device (10) for providing a pressurized flow of breathable gas to a subject during a sleep disordered breathing treatment, the device comprising:
a blower (14) adapted to deliver the pressurized flow of breathable gas via a patient circuit (16) to a patient interface device (18) to be worn by the subject (20) during the sleep disordered breathing treatment;
a nasal congestion detection module (42) adapted to detect a nasal congestion condition of the subject based upon a combination of input parameters determined a predetermined time before delivery or during delivery of the pressurized flow of breathable gas; and
a device settings adaptation module (44) configured to adapt at least one CPAP device setting based on the detection of the condition indicative of nasal congestion, wherein the adaptation of the at least one CPAP device setting contributes to reducing or alleviating nasal congestion during the sleep disordered breathing treatment.

2. The device of claim 1, wherein the nasal congestion detection module (42) comprises at least one selected from the group consisting of:
a voice analysis module (48) adapted to detect the nasal congestion condition from a first input parameter based on an analysis of a voice signal of the subject, wherein the voice signal is captured via a microphone (28,36) coupled to the CPAP device (10),
a mouth breathing detection module (50) adapted to detect the nasal congestion condition from a second input parameter based on an analysis of a measured air flow signal, wherein an unexpected mouth breathing condition determined via the analysis of the measured air flow signal is indicative of the nasal congestion, and
a CPAP usage tracking module (52) adapted to detect the nasal congestion condition from a third input parameter based on a reduced CPAP usage greater than a predetermined CPAP usage over a threshold period of time, wherein the reduced CPAP usage is based on at least one of (a) a measurement of patient interface device (18) wear time ON and wear time OFF and (b) a measurement of sleep disordered breathing treatment continuous use time and discontinuous use time.

3. The device of any one of the preceding claims, wherein the patient interface device (18) comprises a nasal mask (54,64).

4. The device of any one of the preceding claims, further comprising a decongestant agent dispenser (34) configured to dispense a predetermined quantity of at least one nose decongestant agent selected from a predetermined group of nose decongestant agents.

5. The device of claim 4, wherein the at least one CPAP device setting comprises at least a decongestant agent dispenser setting, wherein the decongestant agent dispenser setting is normally set to OFF to not dispense the predetermined quantity of the at least one nose decongestant agent into the pressurized flow of breathable gas via the decongestant agent dispenser in an absence of the detection of the condition indicative of nasal congestion, and set to ON to dispense the predetermined quantity of the at least one nose decongestant agent into the pressurized flow of breathable gas via the decongestant agent dispenser only in response to the detection of the condition indicative of nasal congestion.

6. The device of claim 4 or 5, wherein the predetermined group of nose decongestant agents comprises non-medical agents selected from the group consisting of menthol, eucalyptus, and saline spray.

7. The device of any one of the preceding claims, further comprising a humidifier (32) configured to humidify the pressurized flow of breathable gas, wherein the at least one CPAP device setting comprises a humidifier setting.

8. The device of claim 7, wherein the humidifier setting is normally set to OFF to not moisten the pressurized flow of breathable gas via the humidifier (32) in an absence of the detection of the condition indicative of nasal congestion, and set to ON to moisten the pressurized flow of breathable gas via the humidifier (32) only in response to the detection of the condition indicative of nasal congestion.

9. The device of any one of the preceding claims, further comprising a head position adapter (38) configured to tilt a subject's head towards a more vertical position from a more horizontal position, wherein the head position adapter (38) comprises one or more of an inflatable head pillow adapted to inflate/deflate between the more horizontal position and the more vertical position and a tiltable mattress adapted to tilt between the more horizontal position and the more vertical position, and wherein the at least one CPAP device setting comprises a head position adapter setting.

10. The device of any one of the preceding claims, further comprising a patient interface type recommendation module (48) configured to provide, via an input/output device (26) of the CPAP device (10) in response to a continued detection of the condition indicative of nasal congestion for a period of time greater than a predetermined threshold period of time for at least a single episode of the sleep disordered breathing treatment, a mask type recommendation to be communicated to the subject (20) for advising the subject to wear a mask type other than an only nasal mask type.

11. A method (70) for providing a pressurized flow of breathable gas to a subject during a sleep disordered breathing treatment via a continuous positive airway pressure CPAP device, the method comprising:
delivering (72) the pressurized flow of breathable gas, via a blower and a patient circuit, to a patient interface device to be worn by the subject during the sleep disordered breathing treatment;
detecting (74), via a nasal congestion detection module, a nasal congestion condition of the subject based upon a combination of input parameters determined a predetermined time before delivery or during delivery of the pressurized flow of breathable gas; and
adapting (76), via a device settings adaptation module, at least one CPAP device setting based on the detection of the condition indicative of nasal congestion, wherein the adaptation of the at least one CPAP device setting contributes to reducing or alleviating nasal congestion during the sleep disordered breathing treatment.

12. The method of claim 11, wherein detecting, via the nasal congestion detection module, comprises at least one selected from the group consisting of:
detecting, via a voice analysis module, the nasal congestion condition from a first input parameter based on an analysis of a voice signal of the subject, wherein the voice signal is captured via a microphone coupled to the CPAP device,
detecting, via a mouth breathing detection module, the nasal congestion condition from a second input parameter based on an analysis of a measured air flow signal, wherein an unexpected mouth breathing condition determined via the analysis of the measured air flow signal is indicative of the nasal congestion, and
detecting, via a CPAP usage tracking module, the nasal congestion condition from a third input parameter based on a reduced CPAP usage greater than a predetermined CPAP usage over a threshold period of time, wherein the reduced CPAP usage is based on at least one of (a) a measurement of patient interface device wear time ON and wear time OFF and (b) a measurement of sleep disordered breathing treatment continuous use time and discontinuous use time.

13. The method of claim 11 or 12, further comprising dispensing, via a decongestant agent dispenser, a predetermined quantity of at least one nose decongestant agent selected from a predetermined group of nose decongestant agents, wherein the at least one CPAP device setting comprises at least a decongestant agent dispenser setting, wherein the decongestant agent dispenser setting is normally set to OFF to not dispense the predetermined quantity of the at least one nose decongestant agent into the pressurized flow of breathable gas via the decongestant agent dispenser in an absence of the detection of the condition indicative of nasal congestion, and set to ON to dispense the predetermined quantity of the at least one nose decongestant agent into the pressurized flow of breathable gas via the decongestant agent dispenser only in response to the detection of the condition indicative of nasal congestion.

14. The method of any one of claims 11-13, further comprising humidifying, via a humidifier, the pressurized flow of breathable gas, wherein the at least one CPAP device setting comprises at least a humidifier setting, wherein the humidifier setting is normally set to OFF to not moisten the pressurized flow of breathable gas via the humidifier in an absence of the detection of the condition indicative of nasal congestion, and set to ON to moisten the pressurized flow of breathable gas via the humidifier only in response to the detection of the condition indicative of nasal congestion.

15. The method of any one of claims 11-14, further comprising tilting, via a head position adapter, a subject's head towards a more vertical position from a more horizontal position, wherein the head position adapter comprises one or more of an inflatable head pillow adapted to inflate/deflate between the more horizontal position and the more vertical position and a tiltable mattress adapted to tilt between the more horizontal position and the more vertical position, and wherein the at least one CPAP device setting comprises a head position adapter setting.
